# EUROPEAN PATENT SPECIFICATION

(11) **EP 2 293 840 B1**
(45) Date of publication and mention of the grant of the patent: **04.09.2019**
(21) Application number: 09755257.4
(22) Date of filing: 27.05.2009
(51) Int. Cl.: A61L 15/18, A61L 15/26, A61L 15/28, A61M 1/00, A61F 13/00, A61F 13/02, A61L 15/58

(54) **NEGATIVE PRESSURE WOUND THERAPY DEVICE**
VAKUUM-WUNDTHERAPIEVORRICHTUNG
DISPOSITIF DE TRAITEMENT DE PLAIE À PRESSION NÉGATIVE

(30) Priority: 27.05.2008 US 128957 P
(43) Date of publication of application: 16.03.2011
(73) Proprietor: Smith & Nephew, Inc, Memphis, TN 38116 (US)
(72) Inventor: BUAN, John, Maple Grove MN 55311 (US)
(74) Representative: Smith & Nephew
(86) International application number: PCT/US2009/003232
(87) International publication number: WO 2009/145894

(56) References cited:
- US-A- 4 219 019
- US-A- 4 972 829
- US-A- 5 501 661
- US-A- 5 636 643
- US-A- 5 843 011
- US-A1- 2004 064 132
- US-A1- 2004 241 214
- US-A1- 2006 100 586
- US-A1- 2006 100 586
- US-A1- 2007 265 586
- US-A1- 2008 009 812
- US-A1- 2008 039 759
- US-A1- 2008 108 977

## Description

### TECHNICAL FIELD

The present invention relates, in general, to a device and method for wound therapy that is capable of treating a variety of chronic and acute wound types, including, but not limited to, infection wounds, venous ulcers, arterial ulcers, diabetic ulcers, burn wounds, post amputation wounds, surgical wounds, and the like. Specifically, the present disclosure is related to wound treatment devices and methods that utilize negative pressure therapy.

### BACKGROUND

Negative pressure therapy has been one method used for the treatment of a variety of wounds by practitioners in the art. Conventional negative pressure therapy devices are generally large in size and often require the use of complicated equipment such as suction pumps, vacuum pumps and complex electronic controllers.

Since the negative pressure therapy devices (e.g., dressings) utilize negative pressure, it is desirable to minimize the opportunity for leaks in same, so as to prevent increased damage to the patient and/or wound, or unnecessarily prolonged damage to the patient and/or wound.

Additionally, since negative pressure therapy devices (e.g., dressings) are usually wrapped with, for example, gauze, ace bandages, compression stockings, etc., it would be desirable and beneficial for the connection point between the pump and the negative pressure therapy device to be disposed distally from the negative pressure therapy device. This may increase the comfort of the patient, as well as allow for a limited amount of access to the negative pressure therapy device while it is wrapped.

### BRIEF SUMMARY OF THE INVENTION

The invention is defined by the appended claims.

In accordance with an embodiment of the invention, a wound therapy device includes a backing material, a port hole disposed in the backing material, an absorptive pad disposed on the first side of the backing material such that a portion of the absorptive pad is disposed under the port hole, and, a gasket disposed on the backing material distally between the absorptive pad and the edge.

The use and position of the gasket, along with other factors, are believed to decrease the chances for air pressure leaks. Thus, this will increase the effectiveness and usefulness of the negative pressure therapy device.

In another embodiment of the invention, the gasket is a hydrogel material.

In yet another embodiment of the invention, a wound therapy device also includes a wound interface layer, such as a silver plated mesh, disposed around an exposed portion of the absorptive pad.

In still another embodiment of the invention, a wound therapy device includes a backing material, a port hole disposed in the adhesive backing material, wherein an adaptor is disposed within the port hole and communicating with a connector with a tube having a length such that the connector is distally located from the port hole, an absorptive pad disposed on the first side of the backing material such that a portion of the absorptive pad is disposed under the port hole, and, a gasket disposed distally on the backing material between the absorptive pad and the edge.

By having the connector located distally from the port hole, the patient's comfort can be increased, and access to the connector will not require removal of bandages covering the negative pressure therapy device.

In another aspect of the invention, the invention is a method of making a wound therapy device which includes the steps of applying a port hole to an adhesive substrate, using the port hole as a registration point for applying a gasket material, applying a predetermined thickness of the gasket material in a predetermined shape at a distance around the port hole, applying a liner to the adhesive substrate, and, rolling the adhesive substrate.

In yet another aspect of the invention, the method of making a wound therapy device includes that the gasket material is applied by being poured.

### BRIEF DESCRIPTION OF THE DRAWINGS

The present invention will become more fully apparent from the following description and appended claims, taken in conjunction with the accompanying drawings. Understanding that the accompanying drawings depict only typical embodiments, and are, therefore, not to be considered to be limiting of the scope of the present disclosure, the embodiments will be described and explained with specificity and detail in reference to the accompanying drawings as provided below.
Figure 1 is a cutaway side view of an embodiment of a wound healing device according to the present invention.
Figure 2 is a top view of an embodiment of a wound healing device according to the present invention.
Figure 3 is an exploded view of an embodiment of a wound healing device according to the present invention.
Figure 4 is a back view of an embodiment of a wound healing device according to the present invention.

### DETAILED DESCRIPTION OF THE DRAWINGS

It will be readily understood that the components of the embodiments as generally described and illustrated in the Figures herein could be arranged and designed in a wide variety of different configurations. Thus, the following more detailed description of various embodiments, as represented in the Figures, is not intended to limit the scope of the present disclosure, but is merely representative of various embodiments. While the various aspects of the embodiments are presented in drawings, the drawings are not necessarily drawn to scale unless specifically indicated.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussion of the features and advantages, and similar language, throughout this specification may, but do not necessarily, refer to the same embodiment.

Furthermore, the described features, advantages, and characteristics of the invention may be combined in any suitable manner in one or more embodiments. One skilled in the relevant art will recognize that the invention can be practiced without one or more of the specific features or advantages of a particular embodiment. In other instances, additional features and advantages may be recognized in certain embodiments that may not be present in all embodiments of the invention.

Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

In the following description, numerous specific details are provided to provide a thorough understanding of embodiments of the invention. One skilled in the relevant art will recognize, however, that the invention can be practiced without one or more of the specific details, or with other methods, components, materials, and so forth. In other instances, well-known structures, materials, or operations such as vacuum sources are not shown or described in detail to avoid obscuring aspects of the invention.

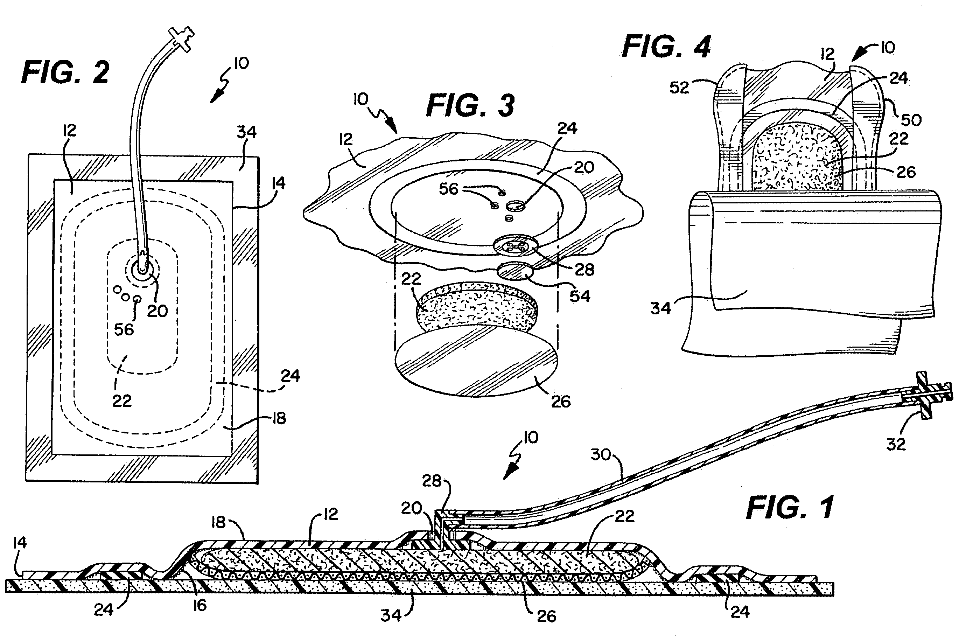

Referring now to Figures 1-4, a wound therapy device 10 is shown. A wound therapy device 10 includes a backing material 12 having a shape with an edge 14 and a first side 16 having an adhesive and a second side 18. The present invention contemplates multiple shapes including, but not limited to, circles, ovals, squares, and oblongs. The backing material 12 may be flexible to allow the device 10 to be contoured to the appropriate location of a wound. In addition, it is preferred that the backing material 12 be semi-permeable. What is meant by the term semi-permeable is that the backing material has breathability aspects that do not impact the ability to hold negative pressure relative to appropriate therapeutic treatment, as would be understood by those of ordinary skill in the art.

It is contemplated that a liner 34 is removably attached to a portion of the first side 16 of the backing material 12. In a preferred embodiment, a second liner 50 and a third liner 52 each disposed on a portion of the edge 14. This is to facilitate quick deploy and use of the device 10. For example, the liner 34 can be removed. The clinician or patient placing the device 10 can utilize second liner 50 and third liner 52 while placing the device 10 without touching the adhesive on the first side 16.

A port hole 20 is disposed in the backing material 18. The port hole 20 can have any shape and size. In an embodiment of the present invention, an adaptor 28 is disposed within the port hole 20. In a preferred embodiment of the invention, a fluid impermeable membrane 54 is disposed on the adaptor 28. The fluid impermeable membrane 54 prohibits fluids and other exudates from flowing from the device 10 to the source of the negative pressure. In a preferred embodiment, the fluid impermeable membrane 54 is GORE-TEX®; however, other materials are contemplated to be used.

A tube segment 30 allows for communication between the adaptor 28 and a connector 32. The connector 32 is connected (either directly or indirectly) to the source of the negative pressure (not shown). The tube segment 30 is long enough that the connector 32 is distally spaced from the adaptor 28 and/or port hole 20. Non-kinking tube material may be used. It is contemplated that the adaptor 28, tube segment 30 and connector 32 is comprised of one structure or multiple structures connected. By moving the connector 32 away from the port hole 20, it is believed that the device will increase the comfort of the patient, since the connection between the device and pump does not have to be located under gauze or other wrapping material such as Unna Boot or COBAN® which typically wraps around the device. Additionally, if the connection between the pump and device needs to be broken, the wrapping material does not need to be removed.

Returning to Figures 1-4, an absorptive pad 22 is disposed adjacent the first side 16 of the backing material 12. The absorptive pad 22 is capable of absorbing exudates and liquid from a wound, while continuing to allow the device to communicate negative pressure to the wound. The absorptive pad 22 may be a material such as sponges, foams, fibers, wicking fibers, hollow fibers, beads, fabrics, or gauzes, super-absorbent materials including super-absorbent polymers in various forms, absorbent foams, gelling agents such as sodium carboxy methyl cellulose, packing materials, and/or combinations thereof. Since the absorptive pad 22 allows the communication of the negative pressure from a pump to the wound, the absorptive pad 22 is disposed under the port hole 20. By used of the term "under," it is meant that when the device 10 is viewed from a top view (such as in Figure 2), a portion of the absorptive pad 22 is disposed beneath the port hole 20. In addition, it is contemplated that the absorptive pad 22 need not be directly beneath or under the port hole 20, *i.e.,* other structures may be disposed between the two structures.

In addition to the port hole 20, it is contemplated that the device 10 includes at least one viewing portal 56 in the backing material 12. Since the absorptive pad 22 retains the exudates and fluids removed from the wound, the viewing portal 56 allows a clinician or patient to determine if the absorptive pad 22 is saturated or not. It is preferred that the viewing portal 56 be disposed between the wound and the port hole 20. In addition, since certain backing materials are non-transparent it is contemplated that a semi-transparent material be disposed over the viewing portal 56 so as to prevent any exudates from leaking out.

It is contemplated that the device 10 also includes wound interface layer 26, or other similar structure, disposed around a portion of the absorptive pad 22. The wound interface layer 26 will allow epithelialization and reduce wound tissue adherence to the device. In one embodiment, the wound interface layer 26 may comprise a silver plated mesh, such as one that is currently commonly available and known as SILVERION®.

A gasket 24 is disposed on the backing material 12, and more particularly on the first side 16 of the backing material 12 with the adhesive. The gasket 24 is disposed distally between the edge 14 of the backing material 12 and the absorptive pad 22. It is contemplated that the gasket 24 be disposed immediately adjacent to the absorptive pad 22.

The gasket 24 has a thickness, and it is contemplated that the thickness of the gasket 24 is between 3 to 5 mils and the width of the gasket 24 is approximately 3/8 of an inch.

In one embodiment of the invention the gasket 24 is a hydrogel. Such materials are currently available from Katecho, in Des Moines, Iowa (USA). It is preferred that the gasket 24 be a material that be biocompatible with skin. In addition the gasket 24 material should mildly adhere to the skin, but not adhere to the skin in the same manner as the adhesive on the backing material. In addition, the gasket 24 material should be mildly flowable. Furthermore, the gasket 24 material should be non-reactive to normal medical device sterility processes. Another contemplated material is a silicone gel; however, it is currently believed to be too cost prohibitive to utilize the silicone gel.

It is preferred that the gasket 24 and the absorptive pad 22 have the same (relatively) shape. It is most preferred that the shape is an oval. Moreover, it is important that the gasket 24 be sized such that the wound is entirely disposed within the gasket 24.

Such a gasket 24 is believed to minimize the possibility of air pressure leaks, and thus increase the efficiency of the device.

In addition to the devices described here, an embodiment of the invention is a method of making a wound therapy device. The method includes the steps of:
applying a port hole to adhesive substrate;
using the port hole as a registration point for applying a gasket material;
applying a predetermined thickness of the gasket material in a predetermined shape at a distance around the port hole;
applying a liner to the adhesive substrate;
rolling the adhesive substrate.

The step of applying a port hole to an adhesive substrate may include punching, cutting, slicing, removing or any other action that results in a port hole being made in an adhesive substrate. The adhesive substrate may be the backing material described above.

By using the port hole as a registration point, it is meant that the port hole location is used as the position to determine where the gasket material is to be applied. Once it is determined where the gasket material is to be applied, the gasket material can be applied at a distance in a predetermined shape around the port hole. It is contemplated that the gasket material is applied by being poured. Additionally, the gasket can be applied in any number of predetermined shapes and thickness.

A liner can be applied to the adhesive substrate and the combination of the adhesive substrate and liner can be rolled and subsequently stored. The resulting combination of adhesive substrate and liner material can then be cut into smaller individual wound therapy devices, and further steps such as providing an absorptive pad and/or providing an adaptor can be accomplished.

It is also contemplated that the method includes the steps of providing the second liner and third liner each on a portion of an edge of the adhesive substrate, such as described above.

Since after use the wound therapy devices usually contain bodily waste typically comprising exudates and liquid from a wound, they are disposable, and thus, decreasing the costs of manufacturing, would be beneficial and is believed to be desirable. Additionally increasing the manufacturability may also be desirable.

Currently, the devices that are described herein are manufactured with a hybrid method, involving some steps that are performed by machine, and other steps that are performed by hand. Such a process may include taking stock material for the gasket and, after cutting the stock material, placing the gaskets by hand onto the backing material. However, it is contemplated that the entire method of manufacture be performed by machines, by hand or a hybrid thereof.

Without further elaboration, it is believed that one skilled in the art can use the preceding description to utilize the present disclosure to its fullest extent The examples and embodiments disclosed herein are to be construed as merely illustrative and not a limitation of the scope of the present disclosure in any way.

## Claims

1. A negative pressure wound therapy device comprising:
a semi-permeable backing material (18) having a shape with an edge (14) and a first side having an adhesive and a second side;
a port hole (20) disposed in the backing material (18) and an adaptor (28) disposed in or within the port hole (20);
an absorptive pad (22) disposed on the first side of the backing material (18) such that a portion of the absorptive pad (22) is disposed under the adaptor (28) and wherein the absorptive pad is capable of absorbing exudates and liquid from a wound, while continuing to allow the device to communicate negative pressure to the wound;
a wound interface layer (26) configured to allow epithelialization and to reduce tissue adherence disposed around a portion of the absorptive pad (22); and,
a gasket (24) disposed on the backing material (18) distally between the absorptive pad (22) and the edge (14).

2. The device of claim 1 wherein the gasket (24) comprises a hydrogel or silicone gel.

3. The device of claim 2 further comprising the absorptive pad (22) having a shape and the gasket (24) has a shape, and wherein the shape of the absorptive pad (22) is substantially the same as the shape of the gasket.

4. The device of claim 3 further comprising the shape of the absorptive pad (22) being an oval.

5. The device of claim 2 further comprising the gasket (24) having a width of approximately 3/8 of an inch.

6. The device of claim 5 further comprising the gasket (24) having a thickness of approximately 30 mils.

7. The device of claim 1 further comprising the gasket (24) being disposed immediately adjacent the absorptive pad (22).

8. The device of any preceding claim wherein the gasket (24) is disposed on the first side of the absorptive pad.

9. The device of claim 1 wherein the wound interface layer (24) is a silver plated mesh.

10. The device of claim 1 further comprising a tubing portion having a first end communicating with the adaptor (28) and second end communicating with a connector.

11. The device of claim 10 wherein the connector is distally spaced from the adaptor (28) and/or the port hole (20).

12. The device of claim 1 wherein the adaptor (28) further comprises a fluid impermeable membrane.

13. The device of claim 1 further comprising a viewing portal disposed in the backing material (18) proximate the adaptor (28).

14. The device of claim 1 further comprising a liner disposed on at least a portion of the adhesive.

15. The device of claim 14 further comprising a second liner disposed on a portion of the edge.

16. The device of claim 1 wherein the shape of the edge (14) of the backing material (18) is oval.

17. The device of any preceding claim wherein the absorptive pad is a super-absorbent material.

18. The device of any preceding claim wherein the absorptive pad comprises a gelling agent.

19. The device of any claims 1 to 16 wherein the absorptive pad is a gauze pad.

## Patentansprüche

1. Eine Unterdruck-Wundtherapievorrichtung, die Folgendes beinhaltet:
ein halbdurchlässiges Trägermaterial (18), das eine Form mit einem Rand (14) und eine erste Seite, die einen Klebstoff aufweist, und eine zweite Seite aufweist;
ein im Trägermaterial (18) angeordnetes Anschlussloch (20) und einen in oder innerhalb des Anschlusslochs (20) angeordneten Adapter (28);
ein Absorptionskissen (22), das auf der ersten Seite des Trägermaterials (18) so angeordnet ist, dass ein Abschnitt des Absorptionskissens (22) unter dem Adapter (28) angeordnet ist, und wobei das Absorptionskissen in der Lage ist, Exsudate und Flüssigkeit aus einer Wunde zu absorbieren, während es der Vorrichtung weiterhin ermöglicht, Unterdruck auf die Wunde zu übertragen;
eine Wundauflageschicht (26), die konfiguriert ist, um Epithelialisierung zu ermöglichen und Gewebehaftung zu reduzieren, angeordnet um einen Abschnitt des Absorptionskissens (22) herum; und,
eine Dichtung (24), die auf dem Trägermaterial (18) distal zwischen dem Absorptionskissen (22) und dem Rand (14) angeordnet ist.

2. Vorrichtung gemäß Anspruch 1, wobei die Dichtung (24) ein Hydrogel oder Silikongel beinhaltet.

3. Vorrichtung gemäß Anspruch 2, die ferner beinhaltet, dass das Absorptionskissen (22) eine Form aufweist und die Dichtung (24) eine Form aufweist, und wobei die Form des Absorptionskissens (22) im Wesentlichen die gleiche wie die Form der Dichtung ist.

4. Vorrichtung gemäß Anspruch 3, die ferner beinhaltet, dass die Form des Absorptionskissens (22) ein Oval ist.

5. Vorrichtung gemäß Anspruch 2, die ferner beinhaltet, dass die Dichtung (24) eine Breite von etwa 3/8 Zoll aufweist.

6. Vorrichtung gemäß Anspruch 5, die ferner beinhaltet, dass die Dichtung (24) eine Dicke von etwa 30 Millizoll aufweist.

7. Vorrichtung gemäß Anspruch 1, die ferner beinhaltet, dass die Dichtung (24) unmittelbar neben dem Absorptionskissen (22) angeordnet ist.

8. Vorrichtung gemäß einem der vorhergehenden Ansprüche, wobei die Dichtung (24) auf der ersten Seite des Absorptionskissens angeordnet ist.

9. Vorrichtung gemäß Anspruch 1, wobei die Wundauflageschicht (24) ein versilbertes Netz ist.

10. Vorrichtung gemäß Anspruch 1, die ferner einen Schlauchabschnitt beinhaltet, der ein erstes Ende, das mit dem Adapter (28) in Verbindung steht, und ein zweites Ende, das mit einem Verbinder in Verbindung steht, aufweist.

11. Vorrichtung gemäß Anspruch 10, wobei der Verbinder distal von dem Adapter (28) und/oder dem Anschlussloch (20) beabstandet ist.

12. Vorrichtung gemäß Anspruch 1, wobei der Adapter (28) ferner eine fluidundurchlässige Membran beinhaltet.

13. Vorrichtung gemäß Anspruch 1, die ferner ein Sichtportal beinhaltet, das in dem Trägermaterial (18) nahe dem Adapter (28) angeordnet ist.

14. Vorrichtung gemäß Anspruch 1, die ferner eine Einlage beinhaltet, die auf mindestens einem Abschnitt des Klebstoffs angeordnet ist.

15. Vorrichtung gemäß Anspruch 14, die ferner eine zweite Einlage beinhaltet, die auf einem Abschnitt des Rands angeordnet ist.

16. Vorrichtung gemäß Anspruch 1, wobei die Form des Rands (14) des Trägermaterials (18) oval ist.

17. Vorrichtung gemäß einem der vorhergehenden Ansprüche, wobei das Absorptionskissen ein superabsorbierendes Material ist.

18. Vorrichtung gemäß einem der vorhergehenden Ansprüche, wobei das Absorptionskissen ein Geliermittel beinhaltet.

19. Vorrichtung gemäß einem der Ansprüche 1 bis 16, wobei das Absorptionskissen ein Gazekissen ist.

## Revendications

1. Un dispositif de thérapie de plaie par pression négative comprenant :
un matériau de support semi-perméable (18) ayant une forme avec un bord (14) et un premier côté ayant un adhésif et un deuxième côté ;
un trou d'orifice (20) disposé dans le matériau de support (18) et un adaptateur (28) disposé dans ou à l'intérieur du trou d'orifice (20) ;
un tampon absorbant (22) disposé sur le premier côté du matériau de support (18) de telle sorte qu'une partie du tampon absorbant (22) soit disposée sous l'adaptateur (28) et le tampon absorbant étant capable d'absorber des exsudats et du liquide d'une plaie, tout en continuant à permettre au dispositif de communiquer une pression négative à la plaie ;
une couche d'interface de plaie (26) conçue pour permettre l'épithélialisation et pour réduire l'adhérence tissulaire disposée autour d'une partie du tampon absorbant (22) ; et,
un joint d'étanchéité (24) disposé sur le matériau de support (18) de manière distale entre le tampon absorbant (22) et le bord (14).

2. Le dispositif de la revendication 1 dans lequel le joint d'étanchéité (24) comprend un hydrogel ou un gel de silicone.

3. Le dispositif de la revendication 2 comprenant en outre le tampon absorbant (22) ayant une forme et le joint d'étanchéité (24) a une forme, et dans lequel la forme du tampon absorbant (22) est substantiellement la même que la forme du joint d'étanchéité.

4. Le dispositif de la revendication 3 comprenant en outre la forme du tampon absorbant (22) qui est un ovale.

5. Le dispositif de la revendication 2 comprenant en outre le joint d'étanchéité (24) ayant une largeur d'environ 3/8 de pouce.

6. Le dispositif de la revendication 5 comprenant en outre le joint d'étanchéité (24) ayant une épaisseur d'approximativement 30 mm.

7. Le dispositif de la revendication 1 comprenant en outre le joint d'étanchéité (24) étant disposé de façon immédiatement adjacente au tampon absorbant (22).

8. Le dispositif de n'importe quelle revendication précédente dans lequel le joint d'étanchéité (24) est disposé sur le premier côté du tampon absorbant.

9. Le dispositif de la revendication 1 dans lequel la couche d'interface de plaie (24) est un maillage plaqué argent.

10. Le dispositif de la revendication 1 comprenant en outre une partie de tubulure ayant une première extrémité en communication avec l'adaptateur (28) et une deuxième extrémité en communication avec un connecteur.

11. Le dispositif de la revendication 10 dans lequel le connecteur est espacé de façon distale de l'adaptateur (28) et/ou du trou d'orifice (20).

12. Le dispositif de la revendication 1 dans lequel l'adaptateur (28) comprend en outre une membrane imperméable aux fluides.

13. Le dispositif de la revendication 1 comprenant en outre un portail de visualisation disposé dans le matériau de support (18) à proximité de l'adaptateur (28).

14. Le dispositif de la revendication 1 comprenant en outre un revêtement disposé sur au moins une partie de l'adhésif.

15. Le dispositif de la revendication 14 comprenant en outre un deuxième revêtement disposé sur une partie du bord.

16. Le dispositif de la revendication 1 dans lequel la forme du bord (14) du matériau de support (18) est ovale.

17. Le dispositif de n'importe quelle revendication précédente dans lequel le tampon absorbant est un matériau super-absorbant.

18. Le dispositif de n'importe quelle revendication précédente dans lequel le tampon absorbant comprend un agent gélifiant.

19. Le dispositif de n'importe quelles revendications 1 à 16 dans lequel le tampon absorbant est un tampon de gaze.
